(19) Europäisches Patentamt
European Patent Office
Office européen des brevets

(11) EP 0 717 037 A1

(12) DEMANDE DE BREVET EUROPEEN

(43) Date de publication:
**19.06.1996 Bulletin 1996/25**

(51) Int Cl.[6]: **C07D 233/54**, A61K 31/415

(21) Numéro de dépôt: **95870132.8**

(22) Date de dépôt: **13.12.1995**

(84) Etats contractants désignés:
**AT BE CH DE DK ES FR GB GR IE IT LI LU MC NL PT SE**

(30) Priorité: **14.12.1994 GB 9425211**

(71) Demandeur: **U C B, S.A.**
**B-1050 Bruxelles (BE)**

(72) Inventeurs:
- **Geerts, Jean-Pierre**
  **B-6860 Léglise (BE)**
- **Motte, Geneviève**
  **B-1450 Chastre (BE)**
- **Differding, Edmond**
  **B-1648 Louvain-la-Neuve (BE)**
- **Henichart, Jean-Pierre**
  **F-59239 La Neuville (FR)**

# (54) 1H-imidazoles substitués ayant une activité alpha 2-agoniste et alpha 1-antagoniste

(57) 4-(1,2,3,4-tétrahydro-1-naphtalényl)-1H-imidazoles et 4-(2,3-dihydro-1H-indén-1-yl)-1H-imidazoles substitués, leurs isomères optiques et leurs mélanges racémiques, leurs sels, leurs procédés de préparation et compositions thérapeutiques les contenant. Ces composés répondent à la formule générale

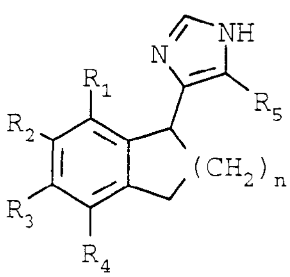

(I)

dans laquelle

n = 1 ou 2,

$R_1$, $R_2$, $R_3$ et $R_4$, qui peuvent être identiques ou différents, représentent chacun un atome d'hydrogène ou d'halogène, un groupe hydroxyle, un radical alkyle ou alkoxy, et

$R_5$ représente un atome d'hydrogène ou un radical alkyle, avec la restriction que $R_1$, $R_2$, $R_3$, $R_4$ et $R_5$ ne peuvent être simultanément de l'hydrogène lorsque n est égal à 2, les radicaux alkyle et alkoxy ayant de 1 à 4 atomes de carbone.

Ce sont des composés nouveaux qui présentent des activités anti-ischémique et anti-hypertensive.

EP 0 717 037 A1

## Description

La présente invention se rapporte à de nouveaux 4-(1,2,3,4-tétrahydro1-naphtalényl)-1H-imidazoles et 4-(2,3-dihydro-1H-indén-1-yl)-1H-imidazoles substitués, à leurs sels d'addition d'acides non toxiques pharmaceutiquement acceptables, à des procédés pour les préparer et à leur utilisation dans le domaine thérapeutique.

Elle concerne également les compositions thérapeutiques renfermant lesdits composés.

K. Matsumoto et al. (203rd ACS National Meeting, Poster n° MEDI-164, San Francisco, 5-10 avril 1992) ont synthétisé le 4-(1,2,3,4-tétrahydro-1-méthyl-1-naphtalényl)-1H-imidazole en un dérivé déméthylé en position 1. Ces composés sont étudiés pour leurs propriétés agonistes des récepteurs $\alpha_2$-adrénergiques. Toutefois, cet article ne mentionne qu'une faible affinité pour les récepteurs $\alpha$-adrénergiques, pour le dérivé méthylé.

Dans le brevet européen 341231 au nom de la demanderesse, on décrit des 1-(1H-imidazol-4-yl)alkyl-benzamides possédant des propriétés anti-ischémiques cardiaques et une puissante activité agoniste des récepteurs $\alpha_2$-adrénergiques pré-synaptiques. Ces composés présentent également une activité diurétique.

Poursuivant ses travaux de recherche dans le domaine, la demanderesse a maintenant synthétisé de nouveaux IH-imidazoles substitués qui présentent de manière surprenante simultanément des propriétés agonistes $\alpha_2$-pré-synaptiques et antagonistes $\alpha_1$-post-synaptiques.

Les composés de la présente invention présentent non seulement d'excellentes propriétés anti-ischémiques cardiaques en rapport avec leurs propriétés $\alpha_2$-aqonistes pré-synaptiques, mais également des propriétés antagonistes des récepteurs $\alpha_1$-adrénergiques leur conférant en plus une propriété anti-hypertensive consécutive à la vasodilatation périphérique.

Ces nouveaux composés peuvent donc être utilisés entre autres pour la prévention et le traitement des troubles induits par l'ischémie en général. Au niveau cardiaque, l'angor est l'expression clinique d'une ischémie myocardique aiguë qui est le résultat d'un déséquilibre momentané entre la demande en oxygène du myocarde et la fourniture en oxygène par la circulation coronaire, déséquilibre pouvant conduire dans les cas graves à l'infarctus du myocarde. C'est pourquoi ces composés conviennent bien pour le traitement de l'angor et de l'infarctus du myocarde. Ces conditions ischémiques pathologiques ont souvent pour cause et pour syndrome l'hypertension artérielle, laquelle représente un facteur aggravant puisque la résistance vasculaire opposée au muscle cardiaque augmente le travail à fournir et amplifie le déséquilibre entre l'apport et la demande en oxygène par la circulation coronaire.

Les composés de l'invention, en plus de leurs effets bénéfiques dans l'ischémie qui sont liés à leurs propriétés agonistes des récepteurs $\alpha_2$-adrénergiques, possèdent également des effets bénéfiques en rapport avec leurs propriétés antagonistes des récepteurs $\alpha_1$-adrénergiques qui sont responsables des effets anti-hypertenseurs observés. La combinaison inattendue de ces deux propriétés apporte aux composés de la présente invention un profil thérapeutique nouveau particulièrement utile dans les conditions d'ischémie s'accompagnant d'hypertension artérielle, que celle-ci en soit la cause ou la conséquence.

Les composés de l'invention trouvent donc leur intérêt dans le traitement des cardiopathies ischémiques hypertensives où jusqu'à présent on doit avoir, le plus souvent, recours à des associations de médicaments telles par exemple les $\beta$-bloquants et les dérivés nitrés ou les dihydropyridines et les dérivés nitrés, ou éventuellement à des composés ou mélanges de composés cumulant deux propriétés complémentaires tel le labétalol ($\alpha$-antagoniste et $\beta$-bloquant).

Les composés de l'invention présentent l'avantage de combiner dans une seule et même molécule les propriétés $\alpha_1$-antagoniste-post-synaptique et $\alpha_2$-agoniste-pré-synaptique.

Outre les effets sur l'ischémie et la tension artérielle, le bénéfice de la combinaison particulière de ce type de propriétés réside dans le fait qu'à l'encontre des $\beta$-bloquants on évite tout risque d'exacerbation de l'asthme chez les sujets sensibles. On évite également les effets néfastes sur le profil lipidique induit par les $\beta$-bloquants. Au contraire, on peut même s'attendre à un effet favorable dans ce domaine tel que cela est rapporté dans la littérature pour les $\alpha_1$-antagonistes sélectifs.

Les nouveaux composés de la présente invention sont des 4-(1,2,3,4-tétrahydro-1-naphtalényl)-1H-imidazole substitués et des 4-(2,3-dihydro-1H-indén-1-yl)-1H-imidazoles substitués répondant à la formule générale

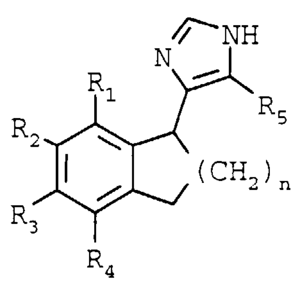

(I)

dans laquelle

n = 1 ou 2,
$R_1$, $R_2$, $R_3$ et $R_4$, qui peuvent être identiques ou différents, représentent chacun un atome d'hydrogène ou d'halogène, un groupe hydroxyle, un radical alkyle ou alkoxy, et
$R_5$ représente un atome d'hydrogène ou un radical alkyle, avec la restriction que $R_1$, $R_2$, $R_3$, $R_4$ et $R_5$ ne peuvent être simultanément de l'hydrogène lorsque n est égal à 2,

les radicaux alkyle et alkoxy ayant de 1 à 4 atomes de carbone, ainsi que leurs sels d'addition d'acides non toxiques pharmaceutiquement acceptables.

Les composés de la présente invention sont des 4-(2,3-dihydro-1H-indén-1-yl)-1H-imidazoles lorsque n est égal à 1; les composés de la présente invention sont des 4-(1,2,3,4-tétrahydro-1-naphtalényl)-1H-imidazoles lorsque n est égal à 2.

La molécule comporte un atome de carbone asymétrique. Les composés de formule I peuvent donc se présenter soit sous la forme racémique, soit sous la forme de l'un ou l'autre énantiomère. Ces diverses formes entrent également dans le cadre de la présente invention.

Les composés préférés conformes à l'invention sont: (+)-4-(1,2,3,4-tétrahydro-6-méthoxy-1-naphtalényl)-1H-imidazole, 4-(1,2,3,4-tétrahydro-5-méthyl-1-naphtalényl)-1H-imidazole, 4-(2,3-dihydro-5-méthoxy-1H-indén-1-yl)-1H-imidazole, (+)-5,6,7,8-tétrahydro-5-(1H-imidazol-4-yl)-2-naphtalénol, 4-(1,2,3,4-tétrahydro-5-méthoxy-1-naphtalényl)-1H-imidazole, 5,6,7,8-tétrahydro-5-(1H-imidazol-4-yl)-1-naphtalénol, 4-(1,2,3,4-tétrahydro-6-méthyl-1-naphtalényl)-1H-imidazole, 2,3-dihydro-1-(1H-imidazol-4-yl)-1H-indène-5-ol, 4-(1,2,3,4-tétrahydro-8-méthoxy-1-naphtalényl)-1H-imidazole, 4-(1,2,3,4-tétrahydro-6,7-diméthoxy-1-naphtalényl)-1H-imidazole, 5,6,7,8-tétrahydro-5-(1H-imidazol-4-yl)-2,3-naphtalènediol.

La présente invention concerne également les sels d'addition d'acides non toxiques pharmaceutiquement acceptables des 4-(1,2,3,4-tétrahydro-1-naphtalényl)-1H-imidazoles et des 4-(2,3-dihydro-1H-indén-1-yl)-1H-imidazoles substitués de formule I. Comme exemples d'acides pharmaceutiquement acceptables, on peut citer les acides minéraux comme les acides chlorhydrique, bromhydrique, sulfurique, nitrique, phosphorique, etc. et les acides organiques comme les acides acétique, citrique, tartrique, benzoïque, salicylique, maléique, etc.

Le procédé général de préparation des 4-(1,2,3,4-tétrahydro-1-naphtalényl)-1H-imidazoles de formule

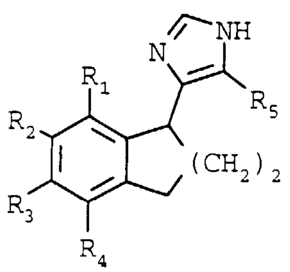

(I)

avec n = 2 dans laquelle $R_1$, $R_2$, $R_3$, $R_4$ représentent chacun un atome d'hydrogène ou d'halogène, un radical alkyle ou alkoxy, les radicaux alkyle et alkoxy ayant de 1 à 4 atomes de carbone, consiste à cycliser un 4-phényl-1-(1-triphénylméthyl-1H-imidazol-4-yl)-1-butanol de formule

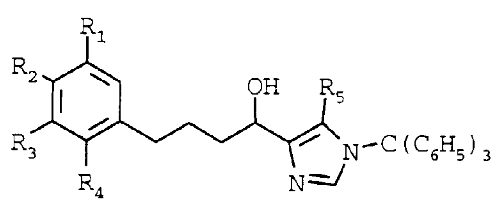

(II)

dans laquelle $R_1$, $R_2$, $R_3$ et $R_4$ ont la signification donnée plus haut et $R_5$ représente un atome d'hydrogène ou un radical alkyle ayant de 1 à 4 atomes de carbone.

Cette cyclisation est généralement effectuée en présence d'un acide organique tel que l'acide formique (qui sert en même temps de solvant) à la température d'ébullition du solvant.

Pour la préparation des 4-(2,3-dihydro-1H-indén-1-yl)-1H-imidazoles substitués de formule

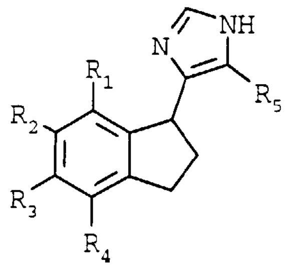

(I)

avec n = 1 dans laquelle $R_1$, $R_2$, $R_3$ et $R_4$ représentent chacun un atome d'hydrogène ou d'halogène, un radical alkyle ou alkoxy, les radicaux alkyle et alkoxy ayant de 1 à 4 atomes de carbone, on soumet un 4-(1H-indén-3-yl)-1H-imidazole de formule

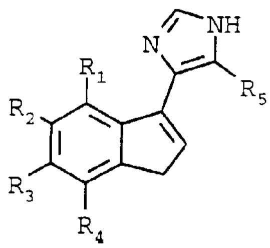

(III)

dans laquelle $R_1$, $R_2$, $R_3$ et $R_4$ ont la signification donnée plus haut et $R_5$ représente un atome d'hydrogène ou un radical alkyle ayant de 1 à 4 atomes de carbone, à une hydrogénation catalytique par de l'hydrogène moléculaire.

Cette réaction est généralement effectuée sous une pression d'hydrogène de 1 à 10 kg en autoclave, dans un solvant, en présence d'un catalyseur tel que du palladium sur carbone, à une température comprise généralement entre 20 et 80 °C.

Suivant encore une autre forme de réalisation, réservée à la préparation de 1H-imidazoles substitués de formule I dans laquelle au moins un des symboles $R_1$, $R_2$, $R_3$ et $R_4$ représente un groupe hydroxyle, on désalkyle sélectivement le (ou les) radical (ou radicaux) alkoxy ayant de 1 à 4 atomes de carbone du IH-imidazole substitué correspondant de formule

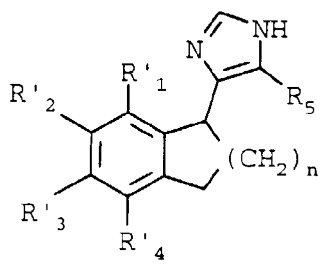

(IV)
(I) avec $R_1$, $R_2$, $R_3$ ou $R_4$ = alkoxy en $C_1$-$C_4$

dans laquelle n=1 ou 2 et $R'_1$, $R'_2$, $R'_3$ et $R'_4$ représentent un atome d'hydrogène ou d'halogène, un radical alkyle ou alkoxy, les radicaux alkyle et alkoxy ayant de 1 à 4 atomes de carbone, au moins un des symboles $R'_1$, $R'_2$, $R'_3$ et $R'_4$ étant un radical alkoxy, et $R_5$ ayant la signification donnée plus haut, en solution dans un solvant, par traitement du composé de formule IV avec de l'acide bromhydrique ou du tribromure de bore ($BBr_3$).

Les sels d'addition d'acides non toxiques pharmaceutiquement acceptables peuvent être préparés à partir des 1H-imidazoles de formule I par des méthodes connues en soi.

Les composés de formule I qui se présentent sous la forme d'un mélange racémique, peuvent être séparés en leurs énantiomères par des méthodes conventionnelles, soit par cristallisation fractionnée des sels diastéréoisomères obtenus par addition au mélange racémique d'un acide optiquement actif, soit par chromatographie du mélange racémique sur un support chiral tel que par exemple une silice sur laquelle est greffée de manière covalente de l'albumine sérique bovine (BSA) ou une phase contenant de l' α-glycoprotéine ou de la β-cyclodextrine. Plusieurs passages successifs sur la colonne chromatographique chirale sont parfois nécessaires pour améliorer la séparation des énantiomères.

Les 4-phényl-1-(1-triphénylméthyl-1H-imidazol-4-yl)-1-butanols de départ de formule II peuvent être préparés à partir des dérivés bromés, convenablement substitués, de formule V et des 1-triphénylméthyl-1H-imidazole-4-carboxaldéhydes de formule VI, par une réaction de Grignard en présence de tournures de magnésium, selon l'équation

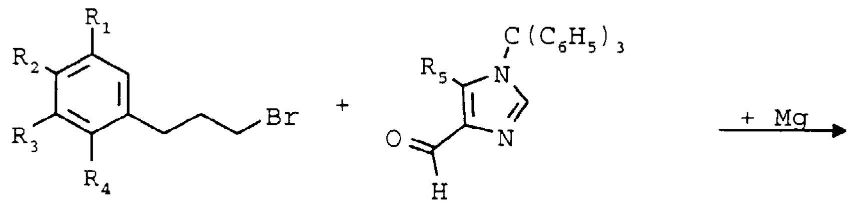


(II)

(V) (VI)

dans ces formules, $R_1$, $R_2$, $R_3$ et $R_4$ représentent chacun un atome d'hydrogène ou d'halogène, un radical alkyle ou alkoxy, les radicaux alkyle et alkoxy ayant de 1 à 4 atomes de carbone, et $R_5$ ayant la signification donnée plus haut.

Les dérivés bromés de formule V utilisés comme produits de départ sont des produits connus ou du commerce.

Les 1-triphénylméthyl-1H-imidazole-4-carboxaldéhydes de formule VI, peuvent être obtenus, de manière générale, par oxydation des 1H-imidazole-4-méthanols correspondants, au moyen de $MnO_2$ activé selon la méthode décrite par J.L. Kelley, C.A. Miller et E.W. McLean (J.Med.Chem.20, (1977), 721-723).

Les 4-(1H-indén-3-yl)-1H-imidazoles de départ de formule III peuvent être préparés par un procédé en plusieurs stades:

(1) condensation de Claisen-Schmidt entre un benzaldéhyde approprié de formule VII et une 1-(1-triphény1méthyl-1H-imidazol-4-yl)-1-éthanone de formule VIII pour obtenir la 3-phényl-1-(1-triphénylméthyl-1H-imidazol-4-yl)-2-propèn-1-one de formule IX selon l'équation

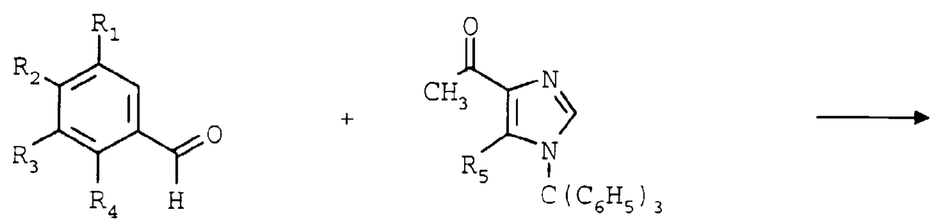

(VII) (VIII)

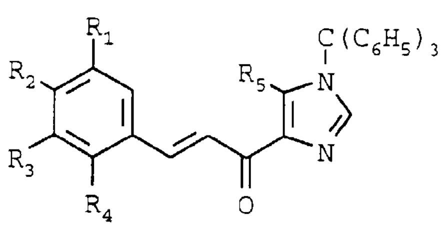

(IX)

(2) hydrogénation sous une pression de 4 kg d'hydrogène en présence d'oxyde de platine de la 3-phényl-1-(1-triphénylméthyl-1H-imidazol-4-yl)-2-propèn-1-one de formule IX en le dérivé 1-propanone correspondant de formule X, selon l'équation

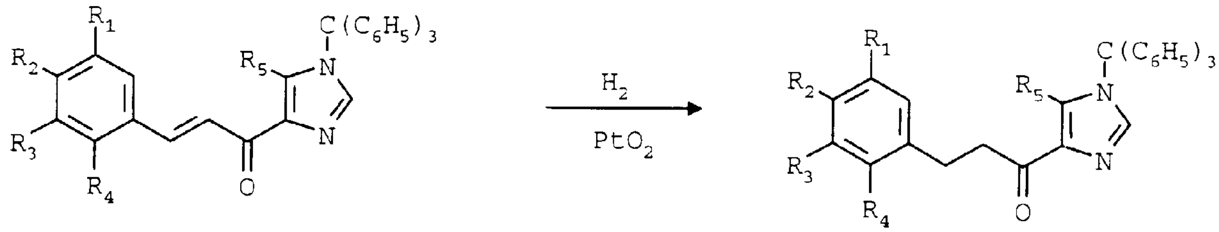


(IX) (X)

(3) suivi d'une détritylation par chauffage dans de l'acide formique du composé de formule X en 1-(1H-imidazol-4-yl)-3-phényl-1-propanone de formule

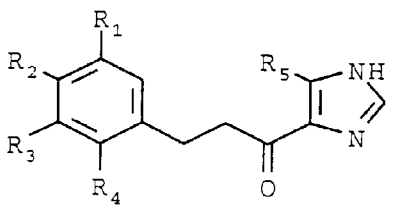

(XI)

(4) cyclisation du composé de formule XI en milieu acide pour obtenir le 4-(1H-indén-3-yl)-1H-imidazole de formule III

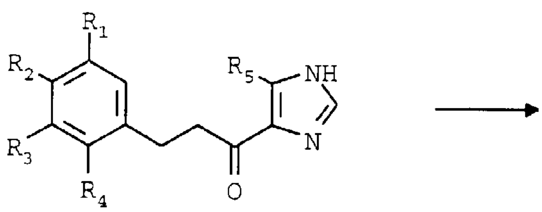

(III)

(XI)

dans ces formules, $R_1$, $R_2$, $R_3$ et $R_4$ représentent chacun un atome d'hydrogène ou d'halogène, un radical alkyle ou alkoxy, les radicaux alkyle et alkoxy ayant de 1 à 4 atomes de carbone et $R_5$ ayant la signification donnée plus haut.

Les 1-(1-triphénylméthyl-1H-imidazol-4-yl)-1-éthanones de formule VIII utilisées comme produit de départ, peuvent être obtenues à partir d'α-méthyl-1-triphénylméthyl-1H-imidazole-4-méthanols qui sont oxydés au moyen de $MnO_2$ selon la méthode décrite par J.L. Kelley et al. (J. Med. Chem. 20 (1977), 721-723; également brevet européen 269599, page 15).

Comme il a été indiqué plus haut, les IH-imidazoles substitués de formule I ainsi que leurs sels d'additions d'acides non toxiques pharmaceutiquement acceptables sont doués de propriétés pharmacologiques de valeur; en particulier, il a été trouvé qu'ils possèdent d'excellentes propriétés anti-ischémiques cardiaques associées à d'utiles propriétés anti-hypertensives.

Les essais pharmacologiques décrits ci-après mettent en évidence ces diverses propriétés.

Les produits suivants de la présente invention ont été soumis à des essais pharmacologiques in vitro et in vivo:

- (+)-4-(1,2,3,4-tétrahydro-6-méthoxy-1-naphtalényl)-1H-imidazole (produit A),
- 4-(1,2,3,4-tétrahydro-5-méthyl-1-naphtalényl)-1H-imidazole (produit B),
- 4-(2,3-dihydro-5-méthoxy-1H-indén-1-yl)-1H-imidazole (produit C),
- (+)-5,6,7,8-tétrahydro-5-(1H-imidazol-4-yl)-2-naphtalénol (produit D),
- 4-(1,2,3,4-tétrahydro-5-méthoxy-1-naphtalényl)-1H-imidazole (produit E),
- chlorhydrate de 5,6,7,8-tétrahydro-5-(1H-imidazol-4-yl)-1-naphtalénol (produit F),
- 4-(1,2,3,4-tétrahydro-6-méthyl-1-naphtalényl)-1H-imidazoie (produit G),
- chlorhydrate de 2,3-dihydro-1-(1H-imidazol-4-yl)-1H-indène-5-ol (produit H),
- 4-(1,2,3,4-tétrahydro-8-méthoxy-1-naphtalényl)-1H-imidazole (produit I),

- 4-(1,2,3,4-tétrahydro-6,7-diméthoxy-1-naphtalényl)-1H-imidazole (produit J), et
- chlorhydrate de 5,6,7,8-tétrahydro-5-(1H-imidazol-4-yl)-2,3naphtalènediol (produit K).

1. Activité anti-ischémique et anti-hypertensive

L'activité anti-ischémique cardiaque des composés se manifeste par leur capacité de s'opposer à l'élévation de l'onde T de l'électrocardiogramme induite par une occlusion coronaire chez le rat. Un suivi continu de la pression artérielle permet également de mettre en évidence les effets anti-hypertenseurs.

L'animal utilisé pour l'essai est un rat albinos mâle de souche Sprague-Dawley, pesant de 220 à 280 g. Le jour de l'expérience, l'animal est anesthésié par administration intrapéritonéale de pentobarbital (60 mg/kg) puis placé sous intubation endotrachéale. Un système de ligature est installé autour de l'artère coronaire antérieure gauche descendante suivant la procédure de Johnston et al. (Can.J.Physiol.Pharmacol., 61 (1983), 1340-1353). Après fermeture du thorax, 3 électrodes sont disposées sur le corps de l'animal (dérivation thoracique "V4"), pour l'enregistrement continu de la forme du signal électrocardiographique (l'onde T notamment). Des cathéters sont introduits dans l'artère carotide droite et la veine jugulaire droite pour la mesure de la pression artérielle et l'administration intraveineuse du composé testé ou de la solution véhicule.

On pratique quatre occlusions coronaires de 45 secondes espacées par des épisodes de reperfusion de 30, 35 et 30 minutes respectivement. Le composé à étudier ou le véhicule sont administrés par voie intraveineuse en bolus, 30 minutes avant la ligature 3. Le signal électrocardiographique T et la pression artérielle sont mesurés en continu pendant toute l'expérience.

Les effets des produits de l'invention sont quantifiés en comparant l'amplitude de l'élévation de l'onde T aux occlusions 3 et 4 (soit 30 minutes et 60 minutes après l'administration) par rapport à sa valeur à l'occlusion 2 (soit avant l'administration). Les résultats sont résumés dans le tableau I suivant où :

- la première colonne indique le produit testé;
- la deuxième colonne indique la dose de produit administrée, exprimée en mole/kg;
- les troisième et quatrième colonnes indiquent l'effet anti-ischémique provoqué par l'administration du composé testé. L'effet anti-ischémique représente la diminution de l'élévation de l'onde T provoquée par les occlusions coronaires 3 et 4, exprimé en % par rapport à l'occlusion 2 antérieure à l'administration du produit (ΔT3 et ΔT4 respectivement) ;
- les cinquième et sixième colonnes indiquent l'effet anti-hypertenseur provoqué par l'administration du composé testé. L'effet anti-hypertenseur est exprimé par la baisse maximale de la tension artérielle mesurée a) entre 5 et 30 minutes et b) entre 30 et 60 minutes après injection intraveineuse du produit testé (ΔPa et ΔPb respectivement). Cette baisse est exprimée en % par rapport à la valeur mesurée avant l'injection.

Comme substances de référence, on utilise les produits suivants:

- oxymétazoline: 3-[(4,5-dihydro-1H-imidazol-2-yl)méthyl]-6-(1,1-diméthyléthyl)-2,4-diméthylphénol, un $\alpha_2$-agoniste pré-synaptique;
- mivazérol: chlorhydrate de 2-hydroxy-3-[(1H-imidazol-4-yl)méthyl]benzamide, un $\alpha_2$-agoniste pré-synaptique (brevet européen 341231);
- prazosine: 1-(4-amino-6,7-diméthoxy-2-quinazolinyl)-4-(2furanylcarbonyl)pipérazine, un $\alpha_1$-antagoniste post-synaptique.
- propranolol: 1-[(1-méthyléthyl)amino]-3-(1-naphtalényloxy)-2-propanol, un agent anti-hypertenseur classique, β-bloquant.

TABLEAU I

| Activité anti-ischémique et anti-hypertensive | | | | | |
|---|---|---|---|---|---|
| Produit | Dose (mole/kg) | ΔT3 (%) | ΔT4 (%) | ΔPa (%) | ΔPb (%) |
| A | $3,2 . 10^{-7}$ | - 80 | - 33 | - 32 | - 28 |
| B | $3,2 . 10^{-8}$ | - 48 | - 29 | - 33 | - 16 |
| C | $3,2 . 10^{-7}$ | - 52 | - 66 | - 44 | - 43 |
| D | $1,0 . 10^{-7}$ | - 50 | - 31 | - 30 | - 17 |
| E | $1,0 . 10^{-7}$ | - 31 | - 13 | - 45 | - 20 |
| F | $3,2 . 10^{-7}$ | - 64 | - 59 | - 32 | - 36 |
| G | $3,2 . 10^{-7}$ | - 24 | - 16 | - 22 | - 14 |

TABLEAU I (suite)

| Activité anti-ischémique et anti-hypertensive | | | | | |
|---|---|---|---|---|---|
| Produit | Dose (mole/kg) | ΔT3 (%) | ΔT4 (%) | ΔPa (%) | ΔPb (%) |
| H | $3{,}2 \cdot 10^{-7}$ | - 48 | - 55 | - 23 | - 19 |
| I | $1{,}0 \cdot 10^{-6}$ | - 37 | - 57 | - 26 | - 16 |
| J | $1{,}0 \cdot 10^{-6}$ | - 31 | - 29 | - 23 | - 16 |
| K | $3{,}2 \cdot 10^{-7}$ | - 49 | - 47 | - 29 | - 41 |
| Oxymétazoline | $3{,}2 \cdot 10^{-8}$ | - 45 | - 38 | 0 | 0 |
| Mivazérol | $3{,}2 \cdot 10^{-8}$ | - 34 | - 22 | - 1 | - 6 |
| Prazosine | $3{,}2 \cdot 10^{-7}$ | NS | NS | - 34 | - 31 |
| Propranolol | $3{,}2 \cdot 10^{-6}$ | - 21 | - 30 | 7 | 5 |
| NS: résultats non significatifs ($p > 0{,}05$) | | | | | |

A l'exception des valeurs de la diminution de l'élévation de l'onde T mesurées pour la prazosine, toutes les valeurs reprises dans le tableau I sont statistiquement significatives ($p < 0{,}05$). De ce tableau, il ressort que les composés de l'invention sont anti-ischémiques ($-13\% \leq \Delta T \leq -80\%$) à des doses comprises entre $10^{-6}$ et $3{,}2\ 10^{-8}$ mole/kg. Cette propriété s'accompagne d'une baisse de la pression artérielle ($-14\% \leq \Delta P \leq -46\%$) aux mêmes doses.
Les substances de référence citées montrent également l'absence d'effet sur la tension artérielle ($0 \leq \Delta P \leq -6\%$) pour des $\alpha_2$-agonistes pré-synaptiques typiques tels que l'oxymétazoline ou le mivazérol et l'absence d'effet anti-ischémique significatif pour l'anti-hypertenseur de type $\alpha_1$-antagoniste tel que la prazosine. Enfin on notera que les produits conformes à l'invention sont plus actifs tant du point de vue de l'activité anti-ischémique que du point de vue de l'effet anti-hypertensif que le propranolol (médicament classique utilisé en clinique humaine pour la thérapie des ischémies cardiaques).

2. Stimulation de l'iléon de cobaye.
Des fragments de muscles longitudinaux attachés à un indicateur de force isométrique, sont plongés dans une solution de Tyrode et sont tendus avec une force de 1 g (G.M. Drew, Brit.J.Pharmacol. 64, (1978), 293-300; M. Andréjak et al., Naunyn-Schmiedeberg's Arch. Pharmacol. 314, (1980), 83-87).
La stimulation électrique des nerfs parasympathiques afférents aux fragments d'iléon provoque une contraction du muscle. Cette contraction est réduite en présence d'un $\alpha_2$-agoniste pré-synaptique et la réduction dépend de la concentration de l'agoniste utilisé. Cet effet est antagonisé par la présence simultanée d'un composé $\alpha_2$-antagoniste tel que l'alpha-yohimbine. Les composés à étudier ont été testés à des concentrations croissantes comprises entre $10^{-10}$ et $10^{-4}$ mole/l.
On détermine la concentration $IC_{30}$ (en mole/l) qui réduit de 30% l'intensité de la contraction du muscle.
Dans le tableau II, on donne les concentrations $IC_{30}$ (en mole/l) obtenues pour les composés de l'invention.

TABLEAU II

| Inhibition de la contraction de l'iléon de cobaye | |
|---|---|
| Produit | $IC_{30}$ (en mole/l) |
| A | $3 \cdot 10^{-8}$ |
| B | $8{,}8 \cdot 10^{-10}$ |
| C | $3{,}2 \cdot 10^{-8}$ |
| D | $1{,}8 \cdot 10^{-9}$ |
| E | $1{,}7 \cdot 10^{-9}$ |
| F | $3{,}7 \cdot 10^{-9}$ |
| G | $3 \cdot 10^{-8}$ |
| H | $3{,}6 \cdot 10^{-8}$ |
| I | $8 \cdot 10^{-6}$ |
| J | $1{,}3 \cdot 10^{-7}$ |
| K | $1{,}6 \cdot 10^{-8}$ |
| oxymétazoline | $1{,}2 \cdot 10^{-8}$ |

En présence d'alpha-yohimbine à la concentration de $10^{-6}$ mole/l, la concentration des composés qui est nécessaire pour réduire de 30% l'intensité de la contraction du muscle est plus élevée et devient supérieure à $10^{-6}$ mole/l, ce qui apporte une confirmation que les composés de l'invention agissent bien au niveau des récepteurs $\alpha_2$-adrénergiques pré-synaptiques. Le tableau II montre également que les composés de l'invention sont au moins aussi actifs, sinon plus, qu'un $\alpha_2$-agoniste pré-synaptique typique tel que l'oxymétazoline.

3. Activité $\alpha_1$-antagoniste post-synaptique.

Une substance $\alpha$-agoniste non sélective comme la norépinéphrine induit une contraction soutenue de l'aorte isolée de rat (J.M. Van Rossum Arch. Int. Pharmacodyn. 143, (1963), 299-330). Cette contraction peut être inhibée par des substances $\alpha_1$-adrénergiques bloquantes comme la prazosine.

Dans ce test, une contraction contrôle au moyen de norépinéphrine à 3,2 . $10^{-8}$ mole/l est effectuée. Après lavage et stabilisation de la préparation, le composé à tester est ajouté au bain. La norépinéphrine est ajoutée 5 minutes après le produit à tester, et l'inhibition de la contraction induite par la norépinéphrine est mesurée. Ensuite, l'inhibition de la contraction induite par la norépinéphrine est évaluée une deuxième et troisième fois en présence de concentrations croissantes du produit testé.

Dans le tableau III ci-dessous, on donne la concentration en mole par litre qui provoque 30% d'inhibition de la contraction induite par la norépinéphrine.

TABLEAU III

| Activité $\alpha_1$-antagoniste post-synaptique | |
|---|---|
| Produit | $ED_{30}$ (mole/litre) |
| A | 2,1 . $10^{-8}$ |
| B | 3,3 . $10^{-8}$ |
| C | 1,0 . $10^{-7}$ |
| D | 4,2 . $10^{-7}$ |
| E | 4,6 . $10^{-7}$ |
| F | 2,3 . $10^{-7}$ |
| G | 7,2 . $10^{-8}$ |
| H | 2,1 . $10^{-7}$ |
| I | 1,8 . $10^{-6}$ |
| J | 2,9 . $10^{-6}$ |
| prazosine | 2,3 . $10^{-10}$ |

Quoique moins actif que la prazosine qui, rappelons-le, n'a pas d'effet anti-ischémique, les produits de l'invention présentent néanmoins des activités $\alpha_1$-antagonistes le plus souvent à des concentrations de l'ordre µmolaire ou moins. Ces activités et les propriétés $\alpha_2$-agonistes pré-synaptiques expliquent la présence simultanée du pouvoir anti-ischémique et anti-hypertenseur des composés de l'invention.

4. Toxicité.

La toxicité des composés de l'invention a été déterminée chez la souris mâle NMRI au moyen du test d'Irwin (S. Irwin, Psychopharmacologia (Berl.), 13, (1968), 222-257).

Des doses progressives du produit étudié sont administrées par voie intrapéritonéale à des groupes de trois souris jusqu'à ce que la dose létale soit atteinte (dose provoquant la mort de deux animaux sur trois dans les 48 heures).

Dans le tableau IV ci-dessous, on donne la dose létale observée pour les composés de l'invention. Il ressort de ce tableau que les composés de l'invention sont très peu toxiques.

TABLEAU IV

| Toxicité | |
|---|---|
| Produit | Dose létale(en mg/kg) |
| A | 228,3 |
| B | > 67,9 |
| C | 214,3 |
| D | > 68,6 |
| E | 127,8 |

TABLEAU IV (suite)

| Toxicité | |
|---|---|
| Produit | Dose létale(en mg/kg) |
| F | 214,3 |
| G | 118,9 |
| H | 236,7 |
| I | 228,3 |
| J | 258,3 |
| K | > 85,4 |

Les compositions pharmaceutiques renfermant les produits de l'invention peuvent être administrées par voie orale, parentérale ou rectale.

Les compositions pharmaceutiques utilisables pour l'administration orale peuvent être solides ou liquides, par exemple sous forme de comprimés (enrobés ou non), pilules, dragées, capsules en gélatine, solutions, sirops, etc.

De même, les compositions utilisables pour l'administration par voie parentérale, sont les formes pharmaceutiquement connues pour ce genre d'administration, par exemple des solutions, des suspensions ou émulsions aqueuses ou huileuses. Pour l'administration par voie rectale, les compositions contenant les produits de l'invention se présentent généralement sous forme de suppositoires.

Les formes pharmaceutiques telles que solutions injectables, suspensions injectables, comprimés, gouttes, suppositoires, etc. sont préparées selon les méthodes couramment utilisées par les pharmaciens.

On mélange les composés de l'invention avec un véhicule solide ou liquide, non toxique, pharmaceutiquement acceptable, et éventuellement avec un agent dispersant, un agent désintégrant, un agent stabilisant, etc. On peut y ajouter, le cas échéant, des agents édulcorants, des agents colorants, etc.

Le pourcentage de produit actif dans les compositions pharmaceutiques peut varier dans des limites très larges, selon le patient et le mode d'administration, en particulier selon la fréquence d'administration.

Quant à la posologie journalière, elle peut varier dans une gamme étendue de doses unitaires selon le mode d'administration. Ainsi, par exemple, elle peut être de 2 à 250 µg de produit actif, une ou deux fois par jour, lorsque l'on administre le composé par voie intraveineuse, ou encore de 20 µg à 5 mg de produit actif une ou deux fois par jour lorsqu'on administre le composé par voie orale.

A titre d'exemple non limitatif d'une composition contenant un composé de l'invention on donne ci-après:

(a) un exemple d'une solution stérile pour injection intraveineuse

| | |
|---|---|
| composé actif | 0,25 mg |
| acétate de sodium | 19,15 mg |
| acid acétique | 3,59 mg |
| chlorure de sodium | 81,0 mg |
| eau pour injectable | ad 10 ml |

(à conserver en ampoule brune de 10 ml, après filtration stérile de la solution)

(b) un exemple de formule pour comprimé

| | |
|---|---|
| composé actif | 0,5 mg |
| amidon de maïs | 38 mg |
| lactose | 63 mg |
| stéarate de magnésium | 1,2 mg |
| polyvinylpyrrolidone | 2,5 ml |

Ci-après, on donne des exemples, non limitatifs, de la préparation des 1H-imidazoles conformes à l'invention ainsi que de leurs composés intermédiaires.

Exemple 1. Préparation des 4-(1,2,3,4-tétrahydro-1-naphtalényl)-1H-imidazoles de formule I (n=2; $R_1$, $R_2$, $R_3$ et $R_4$ = H, halogène ou un radical alkyle ou alkoxy en $C_1$-$C_4$; et $R_5$ = H ou alkyle en $C_1$-$C_4$).

1.1 Préparation des dérivés bromés de départ de formule V.

1.1.a 1-(3-bromopropyl)-2-méthylbenzène.
On prépare ce composé selon la méthode décrite par R.Durand-Dran, dans Ann.Chim.(Paris), 4 (1959), 45-86.
1.1.b 1-(3-bromopropyl)-3-méthylbenzène.
On prépare ce composé selon la méthode décrite par M.T.Bogert et al., dans J.Am.Chem.Soc., 56 (1934), 959-963.
1.1.c 1-(3-bromopropyl)-3-chlorobenzène.
On prépare ce composé selon la méthode décrite par H.König et al., dans Chem.Ber., 92 (1959), 429-433.
1.1.d 1-(3-bromopropyl)-3-(1-méthyléthoxy)benzène.
1.1.e 1-(3-bromopropyl)-2,3-diméthoxybenzène.
1.1.f 1-(3-bromopropyl)-3,4-diméthoxybenzène.
1.1.g 1-(3-bromopropyl)-3-méthoxybenzène.
1.1.h 1-(3-bromopropyl)-2-méthoxybenzène.
1.1.j 1-(3-bromopropyl)-2-chloro-5-méthoxybenzène.
On prépare les composés 1.1.d, 1.1.e, 1.1.f, 1.1.g, 1.1.h et 1.1.j selon la méthode décrite par T.Horaguchi et al., dans J.Het.Chem., 26 (1989), 365-369.

1.2 Préparation des 1-triphénylméthyl-1H-imidazole-4-carboxaldéhydes de départ de formule VI.

1.2.a 1-triphénylméthyl-1H-imidazole-4-carboxaldéhyde.
On prépare ce composé selon la méthode décrite par J.L.Kelley et al. dans J.Med.Chem., 20 (1977), 721-723.
1.2.b 5-méthyl-1-triphénylméthyl-1H-imidazole-4-carboxaldéhyde. On prépare ce composé selon la méthode décrite dans le brevet européen n° 269599 à l'exemple 1.B.3.c.

1.3 Préparation des 4-phényl-1-(1-triphénylméthyl-1H-imidazo1-4-yl)1-butanols de formule II.
On prépare ces composés selon le mode opératoire suivant: on prépare une solution contenant 0,235 mole d'un dérivé bromé préparé à l'exemple 1.1 dans 140 ml d'éther diéthylique sec. On introduit, sous azote, dans un ballon à 4 cols de 4 litres muni d'un agitateur mécanique, d'un thermomètre, d'un réfrigérant à reflux et d'une ampoule à addition à pression constante, 0,25 mole de tournures de magnésium, 140 ml d'éther diéthylique séché sur un alliage sodium/plomb, un cristal d'iode et environ 14 ml (10 %) de la solution de dérivé bromé préparé ci-dessus. On chauffe le mélange à la température du reflux jusqu'au démarrage de la réaction, puis on ajoute progressivement et à un débit tel que l'on maintient le mélange à la température du reflux, le reste (90 %) de la solution de dérivé bromé. On maintient encore 1 heure à la température du reflux, puis on refroidit à la température ambiante et on ajoute, goutte-à-goutte, une solution de 0,23 mole d'un 1-triphénylméthyl-1H-imidazole-4-carboxaldéhyde préparé à l'exemple 1.2 dans 1,25 litre de tétrahydrofuranne sec. On maintient encore le mélange réactionnel à la température ambiante pendant 1 à 4 heures tandis que l'on suit l'évolution de la réaction par chromatographie HPLC. Lorsqu'on estime que la réaction est terminée, on refroidit le mélange réactionnel au bain de glace et on ajoute, goutte-à-goutte, 600 ml d'une solution aqueuse saturée en chlorure d'ammonium. On maintient une demi-heure sous agitation à la température ambiante puis on décante. On lave à trois reprises la phase aqueuse avec 250 ml d'éther diéthylique et on rassemble les phases organiques que l'on sèche ensuite sur sulfate de sodium. On évapore le solvant et on purifie l'huile obtenue par chromatographie HPLC préparative (support: silice de 15 à 40 µm; éluant: mélange 98:2 (v/v) dichlorométhane - méthanol).

1.3.a 4-(2-méthylphényl)-1-(1-triphénylméthyl-1H-imidazol-4-yl)-1butanol.
On obtient ce composé à partir des composés de départ préparés aux exemples 1-1-a et 1.2.a. On recristallise le 4-(2méthylphényl)-1-(1-triphénylméthyl-1H-imidazol-4-yl)-1-butanol obtenu dans l'acétate d'éthyle.
Rendement: 65 %.
P.F.: 125,1°C.

| Analyse pour $C_{33}H_{32}N_2O$ en %: | | | |
|---|---|---|---|
| calculé: | C 83,86 | H 6,83 | N 5,93 |
| trouvé: | 83,68 | 6,91 | 5,99 |

1.3.b 4-(3-méthylphényl)-1-(1-triphénylméthyl-1H-imidazol-4-yl)-1-butanol.
On obtient ce composé à partir des composés de départ préparés aux exemples 1.1.b et 1.2.a. On recristallise le 4-(3-méthylphényl)-1-(1-triphénylméthyl-1H-imidazol-4-yl)-1-butanol obtenu dans l'acétate d'éthyle.
Rendement: 53 %.
P.F.: 105,6°C.

| Analyse pour $C_{33}H_{32}N_2O$ en %: | | | |
|---|---|---|---|
| calculé: | C 83,86 | H 6,83 | N 5,93 |
| trouvé: | 84,00 | 7,07 | 6,08 |

1.3.c 4-(3-chlorophényl)-1-(1-triphénylméthyl-1H-imidazol-4-yl)-1butanol.
On obtient ce composé à partir des composés de départ préparés aux exemples 1.1.c et 1.2.a. On recristallise le 4-(3-chlorophényl)-1-(1-triphénylméthyl-1H-imidazol-4-yl)-1-butanol obtenu dans l'acétate d'éthyle.
Rendement: 45 %.
P.F.: 127,6°C.

| Analyse pour $C_{32}H_{29}ClN_2O$ en %: | | | |
|---|---|---|---|
| calculé: | C 77,96 | H 5,92 | N 5,68 |
| trouvé: | 78,08 | 6,02 | 5,46 |

1.3.d 4-(3-(1-méthyléthoxy)phényl)-1-(1-triphénylméthyl-1H-imidazol-4-yl)-1-butanol.
On obtient ce composé à partir des composés de départ préparés aux exemples 1.1.d et 1.2.a. Le produit est engagé tel quel dans l'étape suivante (exemple 1.4.d).
Rendement: ≈ 25 %.
Spectre de masse: 516 ($M^+$), 498 ($M^+$ - $H_2O$), 273 (cation triphénylméthyl), 243 ($M^+$ - cation triphénylméthyl).

1.3.e 4-(2,3-diméthoxyphényl)-1-(1-triphénylméthyl-1H-imidazol-4-yl)-1butanol.
On obtient ce composé à partir des composés de départ préparés aux exemples 1.1.e et 1.2.a. On recristallise le 4-(2,3-diméthoxyphényl)-1-(1-triphénylméthyl-1H-imidazo1-4-yl)-1-butanol obtenu dans l'éther diéthylique.
Rendement: 32,4 %.
P.F.: 95-99°C.

| Analyse pour $C_{34}H_{34}N_2O_3$ en %: | | | |
|---|---|---|---|
| calculé: | C 78,76 | H 6,56 | N 5,40 |
| trouvé: | 78,47 | 6,58 | 5,47 |

1.3.f 4-(3,4-diméthoxyphényl)-1-(1-triphénylméthyl-1H-imidazol-4-yl)-1-butanol.
On obtient ce composé à partir des composés de départ préparés aux exemples 1.1.f et 1.2.a. On recristallise le 4-(3,4-diméthoxyphényl)-1-(1-triphénylméthyl-1H-imidazol-4-yl)-1-butanol obtenu dans l'acétate d'éthyle.
Rendement: 25,5 %.
P.F.: 145-146°C.

| Analyse pour $C_{34}H_{34}N_2O_3$ en %: | | | |
|---|---|---|---|
| calculé: | C 78,73 | H 6,60 | N 5,40 |
| trouvé: | 78,98 | 6,70 | 5,40 |

1.3.g 4-(3-méthoxyphényl)-1-(1-triphénylméthyl-1H-imidazol-4-yl)-1-butanol.
On obtient ce composé à partir des composés de départ préparés aux exemples 1.1.g et 1.2.a. On recristallise le 4-(3-méthoxyphényl)-1-(1-triphénylméthyl-1H-imidazol-4-yl)-1-butanol obtenu dans l'acétate d'éthyle.
Rendement: 42,4 %.
P.F.: 105-112°C.

| Analyse pour $C_{33}H_{32}N_2O_2$ en %: | | | |
|---|---|---|---|
| calculé: | C 81,15 | H 6,56 | N 5,74 |
| trouvé: | 80,55 | 6,65 | 5,60 |

1.3.h 4-(2-méthoxyphényl)-1-(1-triphénylméthyl-1H-imidazol-4-yl)-1-butanol.
On obtient ce composé à partir des composés de départ préparés aux exemples 1.1.h et 1.2.a. On recristallise le 4-(2-méthoxyphényl)-1-(1-triphénylméthyl-1H-imidazol-4-yl)-1-butanol obtenu dans le toluène.
Rendement: 20 %.
P.F.; 161°C.

| Analyse pour $C_{33}H_{32}N_2O_2$ en %: | | | |
|---|---|---|---|
| calculé: | C 81,11 | H 6,60 | N 5,73 |
| trouvé: | 81,40 | 6,62 | 5,78 |

1.3.i 4-13-méthoxyphényl)-1-15-méthyl-1-triphénylméthyl-1H-imidazol-4-yl)-1-butanol.
On obtient ce composé à partir des composés de départ préparés aux exemples 1.1.g et 1.2.b. On recristallise le 4-(3-méthoxyphényl)-1-(5-méthyl-1-triphénylméthyl-1H-imidazol-4-yl)-1-butanol obtenu dans l'acétate d'éthyle.
Rendement: 7 %.
P.F.: 155°C.

| Analyse pour $C_{34}H_{34}N_2O_2$ en %: | | | |
|---|---|---|---|
| calculé: | C 81,24 | H 6,82 | N 5,57 |
| trouvé: | 80,95 | 6,84 | 5,39 |

1.3.j 4-(2-chloro-5-méthoxyphényl)-1-(1-triphénylméthyl-1H-imidazol-4-yl)-1-butanol.
On obtient ce composé à partir des composés de départ préparés aux exemples 1.1.j et 1.2.a. Le produit est engagé tel quel dans l'étape suivante (exemple 1.4.j).
Rendement: 57 %.

1.4 Cyclisation des 4-phényl-1-(1-triphénylméthyl-1H-imidazol-4-yl)-1-butanols de formule II.
On opère cette cyclisation selon le mode opératoire suivant: on dissout à la température ambiante 1 g d'un 4-phényl-1-(1-triphénylméthyl-1H-imidazol-4-yl)-1-butanol préparé à l'exemple 1.3 dans 10 ml d'acide formique à 99%. On chauffe le mélange réactionnel à la température du reflux pendant 1 à 12 heures tandis que l'on suit l'évolution de la réaction par chromatographie HPLC en phase inverse. Lorsqu'on estime que la réaction est terminée, on distille l'acide formique sous pression réduite et on reprend le résidu de distillation avec un mélange toluène-eau. On décante et on lave la phase aqueuse avec un volume égal de toluène. On alcalinise la phase aqueuse à pH 9 par addition d'une solution aqueuse d'hydroxyde de sodium et on extrait avec du dichlorométhane ou de l'acétate d'éthyle. On sèche la phase organique sur sulfate de sodium et on évapore le solvant sous pression réduite. On obtient une huile qui est purifiée par chromatographie HPLC préparative.

1.4.a 4-(1,2,3,4-tétrahydro-5-méthyl-1-naphtalényl)-1H-imidazole. On obtient ce composé à partir du composé préparé à l'exemple 1.3.a. On laisse le mélange réactionnel à la température du reflux pendant 8 heures. La chromatographie préparative est effectuée dans les conditions suivantes: support: silice; éluant: mélange 97,5:2,5:0,25 (v/v/v) dichlorométhane - méthanol - ammoniaque en solution aqueuse 12 mole/l. Le 4-(1,2,3,4-tétrahydro-5-méthyl-1-naphtalényl)-1H-imidazole finalement obtenu est recristallisé dans l'acétate d'éthyle.
Rendement: 79,4 %
P.F.: 134,9°C.

| Analyse pour $C_{14}H_{16}N_2$ en %: | | | |
|---|---|---|---|
| calculé: | C 79,2 | H 7,6 | N 13,2 |
| trouvé: | 79,15 | 7,85 | 13,55 |

1.4.b 4-(1,2,3,4-tétrahydro-6-méthyl-1-naphtalényl)-1H-imidazole et 4-(1,2,3,4-tétrahydro-8-méthyl-1-naphtalényl)-1H-imidazole. On obtient ces deux isomères à partir du composé préparé à l'exemple 1.3.b. On laisse le mélange réactionnel à la température du reflux pendant 1 heure. La chromatographie préparative est effectuée dans les conditions suivantes: support: silice; éluant: mélange 95:5:0,5 (v/v/v) dichlorométhane -

2-propanol - ammoniaque en solution aqueuse 12 mole/l. Le 4-(1,2,3,4-tétrahydro-6-méthyl-1-naphtalényl)-1H-imidazole est recristallisé dans l'acétate d'éthyle.
Rendement: 8 %
P.F.: 138,8°C.

| Analyse pour $C_{14}H_{16}N_2$ en %: | | | |
|---|---|---|---|
| calculé: | C 79,2 | H 7,6 | N 13,19 |
| trouvé: | 79,14 | 7,99 | 13,22 |

et le 4-(1,2,3,4-tétrahydro-8-méthyl-1-naphtalényl)-1H-imidazole est recristallisé dans un mélange acétate d'éthyle - éther diéthylique.
P.F.: 162°C.

| Analyse pour $C_{14}H_{16}N_2$ en %: | | | |
|---|---|---|---|
| calculé: | C 79,2 | H 7,6 | N 13,19 |
| trouvé: | 79,48 | 7,98 | 13,42 |

1.4.c 4-(6-chloro-1,2,3,4-tétrahydro-1-naphtalényl)-1H-imidazole et 4-(8-chloro-1,2,3,4-tétrahydro-1-naphtalényl)-1H-imidazole. On obtient ces deux isomères à partir du composé préparé à l'exemple 1.3.c. On laisse le mélange réactionnel à la température du reflux pendant 12 heures. La chromatographie préparative est effectuée dans les conditions suivantes: support: silice; éluant: mélange 90:10:5 (v/v/v) acétate d'éthyle - hexane - ammoniaque en solution aqueuse 12 mole/l, dilué à 10% dans l'éthanol. Les produits obtenus sont recristallisés dans l'acétate d'éthyle.
4-(6-chloro-1,2,3,4-tétrahydro-1-naphtalényl)-1H-imidazole:
P.F.: 151,7°C.

| Analyse pour $C_{13}H_{13}ClN_2$ en %: | | | |
|---|---|---|---|
| calculé: | C 67,1 | H 5,63 | N 12,04 |
| trouvé: | 67,0 | 5,7 | 12,59 |

et 4-(8-chloro-1,2,3,4-tétrahydro-1-naphtalényl)-1H-imidazole:
P.F.: 182,9°C.

| Analyse pour $C_{13}H_{13}ClN_2$ en %: | | | |
|---|---|---|---|
| calculé: | C 67,1 | H 5,63 | N 12,04 |
| trouvé: | 67,04 | 5,68 | 12,26 |

1.4.d 4-(1,2,3,4-tétrahydro-6-(1-méthyléthoxy)-1-naphtalényl)-1H-imidazole et 4-(1,2,3,4-tétrahydro-8-(1-méthyléthoxy)-1-naphtalényl)-1H-imidazole.
On obtient ces deux isomères à partir du composé préparé à l'exemple 1.3.d. On laisse le mélange réactionnel à la température du reflux pendant 6 heures. La chromatographie préparative est effectuée dans les conditions suivantes: support: silice; éluant: mélange 96:4:0,5 (v/v/v) dichlorométhane - éthanol - ammoniaque en solution aqueuse 12 mole/l. Les produits obtenus sont recristallisés dans un mélange acétate d'éthyle - éther diéthylique.
4-(1,2,3,4-tétrahydro-6-(1-méthyléthoxy)-1-naphtalényl)-1H-imidazole:
Rendement: 13 %
P.F.: 111,9°C.

| Analyse pour $C_{16}H_{20}N_2O$ en %: | | | |
|---|---|---|---|
| calculé: | C 74,96 | H 7,9 | N 10,93 |
| trouvé: | 74,75 | 8,14 | 10,85 |

et 4-(1,2,3,4-tétrahydro-8-(1-méthyléthoxy)-1-naphtalényl)-1H-imidazole:
P.F.: 133,5°C.

| Analyse pour $C_{16}H_{20}N_2O$ en %: | | | |
|---|---|---|---|
| calculé: | C 74,96 | H 7,9 | N 10,93 |
| trouvé: | 74,93 | 8,14 | 10,91 |

1.4.e 4-(1,2,3,4-tétrahydro-5,6-diméthoxy-1-naphtalényl)-1H-imidazole. On obtient ce composé à partir du composé préparé à l'exemple 1.3.e. On laisse le mélange réactionnel à la température du reflux pendant 3 heures. Le 4-(1,2,3,4-tétrahydro-5,6-diméthoxy-1-naphtalényl)-1H-imidazole est isolé par cristallisation dans l'acétate d'éthyle.
Rendement: 60 %
P.F.: 160°C.

| Analyse pour $C_{15}H_{18}N_2O_2$ en %: | | | |
|---|---|---|---|
| calculé: | C 69,76 | H 6,97 | N 10,85 |
| trouvé: | 70,00 | 7,03 | 10,77 |

1.4.f 4-(1,2,3,4-tétrahydro-6,7-diméthoxy-1-naphtalényl)-1H-imidazole. On obtient ce composé à partir du composé préparé à l'exemple 1.3.f. On laisse le mélange réactionnel à la température du reflux pendant 3 heures. Le 4-(1,2,3,4-tétrahydro-6,7-diméthoxy-l-naphtalényl)-lH-imidazole est obtenu par cristallisation dans l'acétate d'éthyle.
Rendement: 73 %
P.F.: 140°C.

| Analyse pour $C_{15}H_{18}N_2O_2$ en %: | | | |
|---|---|---|---|
| calculé: | C 69,76 | H 6,97 | N 10,85 |
| trouvé: | 69,30 | 7,41 | 10,50 |

1.4.g 4-(1,2,3,4-tétrahydro-6-méthoxy-1-naphtalényl)-1H-imidazole et 4-(1,2,3,4-tétrahydro-8-méthoxy-1-naphtalényl)-1H-imidazole. On obtient ces deux isomères à partir du composé préparé à l'exemple 1.3.g. On laisse le mélange réactionnel à la température du reflux pendant 3 heures. La chromatographie préparative est effectuée dans les conditions suivantes: support: silice; éluant: mélange 97,5:2,5 (v/v) dichlorométhane - méthanol. Le 4-(1,2,3,4-tétrahydro-6-méthoxy-1-naphtalényl)-1H-imidazole est recristallisé dans l'acétate d'éthyle:
Rendement: 33 %
P.F.: 160-165°C.

| Analyse pour $C_{14}H_{16}N_2O$ en %: | | | |
|---|---|---|---|
| calculé: | C 73,65 | H 7,06 | N 12,27 |
| trouvé: | 73,70 | 7,05 | 12,27 |

et le 4-(1,2,3,4-tétrahydro-8-méthoxy-1-naphtalényl)-1H-imidazole est recristallisé dans l'éther diéthylique:
Rendement: 46 %
P.F.: 135-140°C.

| Analyse pour $C_{14}H_{16}N_2O$ en %: | | | |
|---|---|---|---|
| calculé: | C 73,65 | H 7,06 | N 12,27 |
| trouvé: | 73,30 | 7,12 | 12,27 |

1.4.h 4-(1,2,3,4-tétrahydro-5-méthoxy-1-naphtalényl)-1H-imidazole. On obtient ce composé à partir du composé préparé à l'exemple 1.3.h. On laisse le mélange réactionnel à la température du reflux pendant 2 heures. La chromatographie préparative est effectuée dans les conditions suivantes: support: silice; éluant: mélange 95:5 (v/v) dichlorométhane - méthanol. Le 4-(1,2,3,4-tétrahydro-5-méthoxy-1-naphtalényl)-1H-imidazole obtenu est recristallisé dans le toluène.
Rendement: 20 %
P.F.: 156°C.

| Analyse pour $C_{14}H_{16}N_2O$ en %: | | | |
|---|---|---|---|
| calculé: | C 73,65 | H 7,06 | N 12,27 |
| trouvé: | 73,43 | 7,09 | 12,10 |

1.4.i 5-méthyl-4-(1,2,3,4-tétrahydro-6-méthoxy-1-naphtalényl)-1H-imidazole et 5-méthyl-4-(1,2,3,4-tétrahydro-8-méthoxy-1-naphtalényl)-1H-imidazole.

On obtient ces deux isomères à partir du composé préparé à l'exemple 1.3.i. On laisse le mélange réactionnel à la température du reflux pendant 2 heures. La chromatographie préparative est effectuée dans les conditions suivantes: support: silice; éluant: mélange 97:3 (v/v) dichlorométhane - méthanol. Le 5-méthyl-4-(1,2,3,4-tétrahydro-6-méthoxy-1-naphtalényl)-1H-imidazole est recristallisé dans le 2-propanol:

P.F.: 216°C.

| Analyse pour $C_{15}H_{18}N_2O$ en %: | | | |
|---|---|---|---|
| calculé: | C 74,34 | H 7,49 | N 11,56 |
| trouvé: | 74,01 | 7,58 | 11,22 |

et le 5-méthyl-4-(1,2,3,4-tétrahydro-8-méthoxy-1-naphtalényl)-1H-imidazole est recristallisé dans un mélange 2-propanol - di(1-méthy1éthyl)éther : P.F.: 135°C.

| Analyse pour $C_{15}H_{18}N_2O$ en %: | | | |
|---|---|---|---|
| calculé: | C 74,34 | H 7,49 | N 11,56 |
| trouvé: | 73,61 | 7,70 | 11,36 |

1.4.j 4-15-chloro-1,2,3,4-tétrahydro-8-méthoxy-1-naphtalényl)-1H-imidazole.

On obtient ce composé à partir du composé préparé à l'exemple On laisse le mélange réactionnel à la température du reflux pendant 3 heures. On extrait le 4-(5-chloro-1,2,3,4-tétrahydro-8-méthoxy-1-naphtalényl)-1H-imidazole du milieu réactionnel avec du chloroforme et on le recristallise dans ce solvant.

Rendement: 81 %

P.F.: 204°C.

| Analyse pour $C_{14}H_{15}ClN_2O$ en %: | | | |
|---|---|---|---|
| calculé: | C 64,0 | H 5,75 | N 10,66 |
| trouvé: | 63,82 | 5,82 | 10,53 |

1.5 Préparation de 4-(1,2,3,4-tétrahydro-1-naphtalényl)-1H-imidazoles optiquement actifs.

(+)-4-(1,2,3,4-tétrahydro-6-méthoxy-1-naphtalényl)-1H-imidazole et (-)-4-(1,2,3,4-tétrahydro-6-méthoxy-1-naphtalényl)-1H-imidazole.

Le 4-(1,2,3,4-tétrahydro-6-méthoxy-1-naphtalényl)-1H-imidazole racémique préparé à l'exemple 1.4.g est dédoublé par chromatographie sur support chiral (CHIRACEL OJ de la société DAICEL: para-méthylbenzoate de cellulose) avec comme éluant un mélange 80:20:0,1 (v/v/v) hexane - 2-propanol - diéthylamine. Le (+)-4-(1,2,3,4-tétrahydro-6-méthoxy-1-naphtalényl)-1H-imidazole est élué le premier:

Rendement après recristallisation dans l'acétate d'éthyle: 66 %

P.F.: 183°C.

$[\alpha]_D^{25}$: + 13,85° (c=1, méthanol)

Le (-)-4-(1,2,3,4-tétrahydro-6-méthoxy-1-naphtalényl)-1H-imidazole est élué ensuite:

Rendement après recristallisation dans l'acétate d'éthyle: 62 %

P.F.: 183°C.

$[\alpha]_D^{25}$: - 14,0° (c=1, méthanol)

Exemple 2. Préparation des 4-(2,3-dihydro-1H-indén-1-yl)-1H-imidazoles de formule I (n=1; $R_1$, $R_2$, $R_3$ et $R_4$ = H, halogène ou un radical alkyle ou alkoxy en $C_1$-$C_4$; et R5 = H ou alkyle en $C_1$-$C_4$).

2.1 Préparation des 1-(1-triphénylméthyl-1H-imidazol-4-yl)-1-éthanones de départ de formule VIII.

1-(1-triphénylméthyl-1H-imidazol-4-yl)-1-éthanone.

On prépare ce composé selon la méthode décrite dans le brevet européen n° 269599 à l'exemple 1.B.4.

2.2 Préparation des 3-phényl-1-(1-triphénylméthyl-1H-imidazol-4-yl)-2-propèn-1-ones de formule IX.

2.2.a 3-(2,3-diméthoxyphényl)-1-(1-triphénylméthyl-1H-imidazol-4-yl)-2-propèn-1-one.
On introduit 0,149 mole de 2,3-diméthoxybenzaldéhyde en 5 heures et à la température ambiante dans une solution de 0,149 mole de 1-(1-triphénylméthyl-1H-imidazol-4-yl)-1-éthanone préparée à l'exemple 2.1 dans un mélange de 800 ml d'éthanol et de 186 ml d'une solution aqueuse d'hydroxyde de sodium 2N. Le milieu réactionnel est ensuite amené à pH 7,5 par addition d'une solution aqueuse d'acide chlorhydrique 5N. On distille l'éthanol et il se forme un solide qui précipite. On filtre ce solide et on le lave avec de l'eau avant de l'essorer. Le solide est redissous dans 2 litres de 2-propanol à la température d'ébullition et on filtre les sels insolubles. Le 2-propanol est évaporé à moitié et on recristallise la 3-(2,3-diméthoxyphényl)-1-(1-triphénylméthyl-1H-imidazol-4-yl)-2-propèn-1-one. Rendement: 75 %.
P.F.: 175-186°C.

| Analyse pour $C_{33}H_{28}N_2O_3$en %: | | | |
|---|---|---|---|
| calculé: | C 79,2 | H 5,60 | N 5,6 |
| trouvé: | 78,25 | 5,70 | 5,37 |

2.2.b 3-(3-méthoxyphényl)-1-(1-triphénylméthyl-1H-imidazol-4-yl)-2-propèn-1-one.
On prépare ce composé selon le mode opératoire décrit à l'exemple 2.2.a au départ du 3-méthoxybenzaldéhyde. Rendement: 98 %.
P.F.: 173-175°C.

| Analyse pour $C_{32}H_{26}N_2O_2$ en %: | | | |
|---|---|---|---|
| calculé: | C 81,68 | H 5,56 | N 5,95 |
| trouvé: | 81,65 | 5,62 | 5,99 |

2.3 Préparation des 3-phényl-1-(1-triphénylméthyl-1H-imidazol-4-yl)-1-propanones de formule X.

2.3.a 3-(2,3-diméthoxyphényl)-1-(1-triphénylméthyl-1H-imidazol-4-yl)-1-propanone.
On hydrogène sous 4 kg de pression en hydrogène et en présence de 3 g d'oxyde de platine à 20°C, 0,108 mole de 3-(2,3-diméthoxyphényl)-1-(1-tripheny1méthyl-1H-imidazol-4-yl)-2-propèn-1-one préparée à l'exemple 2.2.a dans 800 ml d'acide acétique. En fin de réaction, on filtre le catalyseur et on évapore l'acide acétique sous pression réduite. On purifie le résidu par chromatographie (support: silice; éluant: mélange 99:1:0,1 (v/v/v) dichlorométhane - méthanol - ammoniaque en solution aqueuse 12 mole/l). On isole d'abord la 3-(2,3-diméthoxyphényl)-1-(1-triphénylméthyl-1H-imidazol-4-yl)-1-propanone qui est utilisée telle quelle dans l'étape suivante (exemple 2.4.a), et en poursuivant la chromatographie (éluant: mélange 95:5:0,5 (v/v/v) dichlorométhane - méthanol - ammoniaque en solution aqueuse 12 mole/l) on isole ensuite la 3-(2,3-diméthoxyphényl)-1-(1H-imidazol-4-yl)-1-propanone qui est également utilisée telle quelle dans l'étape suivante (exemple 2.5.a).

2.3.b 3-(3-méthoxyphényl)-1-(1-triphénylméthyl-1H-imidazol-4-yl)-1-propanone.
On obtient ce composé selon le mode opératoire décrit à l'exemple 2.3.a. On isole par chromatographie d'abord la 3-(3-méthoxyphényl)-1-(1-triphénylméthyl-1H-imidazol-4-yl)-1-propanone qui est utilisée telle quelle dans l'étape suivante (exemple 2.4.b), et en poursuivant la chromatographie, on isole ensuite la 3-(3-méthoxyphényl)-1-(1H-imidazol-4-yl)-1-propanone qui est cristallisée dans l'acétate d'éthyle avant d'être engagée dans l'étape suivante (exemple 2.5.b).

2.4 Préparation des 1-(1H-imidazol-4-yl)-3-phényl-1-propanones de formule XI.

2.4.a 3-(2,3-diméthoxyphényl)-1-(1H-imidazol-4-yl)-1-propanone. On chauffe pendant 0,5 heure et à 80°C, 17,88 g (0,0687 mole) de 3-(2,3-diméthoxyphényl)-1-(1-triphénylméthyl-1H-imidazol-4-yl)-1-propanone préparée à l'exemple 2.3.a dans 100 ml d'acide formique. On refroidit ensuite le milieu réactionnel jusqu'à la température ambiante et on filtre le triphénylméthanol qui a précipité pendant le refroidissement. On distille l'acide formique et on reprend le résidu dans de l'eau. La phase aqueuse est ensuite alcalinisée à pH 9 par

addition d'une solution aqueuse d'ammoniaque 12 mole/1. On filtre et on sèche le précipité qui s'est formé et on obtient 8,35 g de 3-(2,3diméthoxyphényl)-1-(1H-imidazol-4-yl)-1-propanone.
Rendement: 90 %.
P.F.: 120-125°C.

| Analyse pour $C_{14}H_{16}N_2O_3$ en %: | | | |
|---|---|---|---|
| calculé: | C 64,6 | H 6,2 | N 10,76 |
| trouvé: | 64,04 | 6,21 | 10,33 |

2.4.b 3-(3-méthoxyphényl)-1-(1H-imidazol-4-yl)-1-propanone. Selon le même mode opératoire qu'à l'exemple 2.4.a et au départ de 10,28 g (0,0217 mole) de 3-(3-méthoxyphényl)-1-(1-triphénylméthyl-1H-imidazol-4-yl)-1-propanone préparée à l'exemple 2.3.b, on obtient, après cristallisation dans l'acétate d'éthyle, 3,68 g de 3-(3-méthoxyphényl)-1-(1H-imidazol-4-yl)-1-propanone.
Rendement: 73 %.
P.F.: 127-129°C.

| Analyse pour $C_{13}H_{14}N_2O_2$ en %: | | | |
|---|---|---|---|
| calculé: | C 67,82 | H 6,08 | N 12,17 |
| trouvé: | 67,18 | 6,10 | 11,70 |

2.5 Préparation des 4-(1H-indén-3-yl)-1H-imidazole de formule III.

2.5.a 4-(6,7-diméthoxy-1H-indén-3-yl)-1H-imidazole. On agite à température ambiante et pendant 16 heures, 5 g (0,0206 mole) de 3-(2,3-diméthoxyphényl)-1-(1H-imidazol-4-yl)-1-propanone préparée à l'exemple 2.4.a (et/ou à l'exemple 2.3.a), dans 100 ml d'un mélange 50:50 (v/v) d'acide phosphorique à 85% et d'acide sulfurique à 95%. On verse ensuite le milieu réactionnel sur de la glace, on alcalinise le mélange à pH 7,5 par addition d'une solution aqueuse d'hydroxyde de sodium refroidie à une température inférieure à 10°C, puis on extrait le mélange avec du dichlorométhane. On évapore le solvant de la phase organique puis on engage le résidu dans une chromatographie (support: silice; éluant: mélange 95:5 (v/v) dichlorométhane - méthanol). On obtient 2,3 g de 4-(6,7-diméthoxy-1H-indén-3-yl)-1H-imidazole.
Rendement: 41 %
P.F. : 150 °C.

2.5.b 4-(6-méthoxy-1H-indén-3-yl)-1H-imidazole.
Selon le même mode opératoire qu'à l'exemple 2.5.a et au départ de 12,93 g (0,0562 mole) de 3-(3-méthoxyphényl)-1-(1H-imidazol-4-yl)-1-propanone préparée à l'exemple 2.4.b (et/ou à l'exemple 2.3.b), on obtient, après cristallisation dans l'acétonitrile, 3,76 g de 4-(6-méthoxy-1H-indén-3-yl)-1H-imidazole.
Rendement: 31,5 %.
P.F.: 170°C.

| Analyse pour $C_{13}H_{12}N_2O$ en %: | | | |
|---|---|---|---|
| calculé: | C 73,56 | H 5,70 | N 13,20 |
| trouvé: | 73,50 | 5,70 | 13,23 |

2.6 Hydrogénation des 4-(1H-indén-3-yl)-1H-imidazole de formule III.

2.6.a 4-(2,3-dihydro-4,5-diméthoxy-1H-indén-1-yl)-1H-imidazole.
On hydrogène sous 2 kg de pression en hydrogène, en présence de palladium sur carbone à 10% et à 40°C, 2,3 g (0,0095 mole) de 4-(6,7-diméthoxy-lH-indén-3-yl)-lH-imidazole préparé à l'exemple 2.5.a dans 200 ml de méthanol. En fin de réaction, on filtre le catalyseur et on distille le méthanol. On cristallise le résidu dans l'acétate d'éthyle et on obtient le 4-(2,3-dihydro-4,5-diméthoxy-1H-indén-1-yl)-1H-imidazole.
P.F.: 156-158°C.

| Analyse pour $C_{14}H_{16}N_2O_2$ en %: |
|---|

(suite)

| | | | |
|---|---|---|---|
| calculé: | C 68,83 | H 6,60 | N 11,47 |
| trouvé: | 68,94 | 6,63 | 11,34 |

2.6.b 4-(2,3-dihydro-5-méthoxy-1H-indén-1-yl)-1H-imidazole.
On prépare ce composé selon le mode opératoire décrit à l'exemple 2.6.a au départ de 4-(6-méthoxy-1H-indén-3-yl)-1H-imidazole préparé à l'exemple 2.5.b.
Rendement: 81,2 %.
P.F.: 180-182°C.

| Analyse pour $C_{13}H_{14}N_2O$ en %: | | | |
|---|---|---|---|
| calculé: | C 72,89 | H 6,54 | N 13,08 |
| trouvé: | 73,14 | 6,58 | 12,68 |

Exemple 3. Préparation des 5,6,7,8-tétrahydro-5-(1H-imidazol-4-yl)-naphtalénols et des 2,3-dihydro-1-(1H-imidazol-4-yl)-1H-indénols de formule I (n=1 ou 2; $R_1$, $R_2$, $R_3$ et/ou $R_4$=OH; et $R_5$ = H ou alkyle en $C_1$-$C_4$).

On prépare ces composés selon le mode opératoire suivant: on chauffe pendant une demi-heure 1 g d'un 4-(1,2,3,4-tétrahydro-1-naphtalényl)-1H-imidazole ou d'un 4-(2,3-dihydro-1H-indén-1-yl)-1H-imidazole portant au moins un radical alkoxy ayant de 1 à 4 atomes de carbone, dans 20 ml d'acide bromhydrique azéotropique à la température d'ébullition. On distille ensuite l'acide bromhydrique sous pression réduite, on reprend le résidu dans de l'eau et on alcalinise à pH 10 par addition d'une solution aqueuse d'hydroxyde de sodium. On distille l'eau sous pression réduite et on extrait le résidu avec du 2-propanol à la température d'ébullition. On filtre les sels insolubles et on évapore partiellement le solvant jusqu'à apparition de cristaux. Dans le cas où ces composés ne peuvent être isolés de la sorte, on forme le chlorhydrate correspondant selon le mode opératoire suivant: on dissout de l'acide chlorhydrique gazeux dans du 2propanol et on introduit le composé brut formé auparavant dans cette solution. On filtre le précipité qui s'est formé et on le purifie par recristallisation si nécessaire.

3.a (+)-5,6,7,8-tétrahydro-5-(1H-imidazol-4-yl)-2-naphtalénol.
On obtient 0,7 g de (+)-5,6,7,8-tétrahydro-5-(1H-imidazol-4-yl)2-naphtalénol au départ de 1,5 g de (+)-4-(1,2,3,4-tétrahydro-6-méthoxy-1-naphtalényl)-1H-imidazole préparé à l'exemple 1.5.
P.F.: 243-246°C.
$[\alpha]_D^{25}$: +5,11° (c=1; méthanol)

| Analyse pour $C_{13}H_{14}N_2O$ en %: | | | |
|---|---|---|---|
| calculé: | C 72,87 | H 6,58 | N 13,07 |
| trouvé: | 72,23 | 6,80 | 12,64 |

3.b (-)-5,6,7,8-tétrahydro-5-(1H-imidazol-4-yl)-2-naphtalénol.
On obtient 0,6 g de (-)-5,6,7,8-tétrahydro-5-(1H-imidazo1-4-yl)-2-naphtalénol au départ de 1,5 g de (-)-4-(1,2,3,4-tétrahydro-6-méthoxy-1-naphtalényl)-1H-imidazole préparé à l'exemple 1.5.
P.F.: 243-246°C.
$[\alpha]_D^{25}$: -5,5° (c=1; méthanol)

3.c Chlorhydrate de 5,6,7,8-tétrahydro-5-(1H-imidazol-4-yl)-2,3-naphtalènediol.
On obtient 0,63 g de chlorhydrate de 5,6,7,8-tétrahydro-5-(1H-imidazol-4-yl)-2,3-naphtalènediol au départ de 1,5 g de 4-(1,2,3,4-tétrahydro-6,7-diméthoxy-1-naphtalényl)-1H-imidazole préparé à l'exemple 1.4.f.
P.F.: 238-242°C.

| Analyse pour $C_{13}H_{14}N_2O_2$.HCl en %: | | | |
|---|---|---|---|
| calculé: | C 58,54 | H 5,67 | N 10,50 |

(suite)

| | | | |
|---|---|---|---|
| trouvé: | 57,21 | 5,73 | 10,17 |

3.d Chlorhydrate de 5,6,7,8-tétrahydro-5-(1H-imidazol-4-yl)-1,2-naphtalènediol.
On obtient, après recristallisation dans un mélange 2-propanol - di(1-méthyléthyl)éther, 6,93 g de chlorhydrate de 5,6,7,8-tétrahydro-5-(1H-imidazol-4-yl)-1,2-naphtalènediol au départ de 10,18 g de 4-(1,2,3,4-tétrahydro-5,6-diméthoxy-1-naphtalényl)-1H-imidazole préparé à l'exemple 1.4.e.
Rendement: 66 %.
P.F.: 245-250°C.

| Analyse pour $C_{13}H_{14}N_2O_2$.HCl en %: | | | | |
|---|---|---|---|---|
| calculé: | C 58,53 | H 5,63 | N 10,5 | $Cl^-$ 13,32 |
| trouvé: | 58,47 | 5,66 | 10,53 | 12,86 |

3.e Chlorhydrate de 5,6,7,8-tétrahydro-5-(1H-imidazo1-4-yl)-1-naphtalénol.
On obtient, après recristallisation dans un mélange 2-propanol - di(1-méthyléthyl)éther, 1,28 g du chlorhydrate de 5,6,7,8-tétrahydro-5-(1H-imidazol-4-yl)-1-naphtalénol au départ de 1,66 g de 4-(1,2,3,4-tétrahydro-5-méthoxy-1-naphtalényl)-1H-imidazole préparé à l'exemple 1.4.h.
Rendement: 85 %
P.F.: 215-222°C.

| Analyse pour $C_{13}H_{14}N_2O$.HCl en %: | | | |
|---|---|---|---|
| calculé: | C 62,27 | H 6,03 | N 11,17 |
| trouvé: | 60,36 | 6,07 | 10,39 |

3.f Chlorhydrate de 2,3-dihydro-1-(1H-imidazol-4-yl)-1H-indène-4,5-diol.
On obtient 0,5 g de chlorhydrate de 2,3-dihydro-1-(1H-imidazol-4-yl)-1H-indène-4,5-diol au départ de 0,4 g de 4-(2,3-dihydro-4,5-diméthoxy-1H-indén-1-yl)-1H-imidazole préparé à l'exemple 2.6.a. Rendement: 83 % P.F.: 240-245°C.

| Analyse pour $C_{12}H_{12}N_2O_2$.HCl en %: | | | |
|---|---|---|---|
| calculé: | C 57,04 | H 5,19 | N 11,09 |
| trouvé: | 56,96 | 4,84 | 10,93 |

3.g Chlorhydrate de 2,3-dihydro-1-(1H-imidazol-4-yl)-1H-indène-5-ol.
On obtient 0,36 g de chlorhydrate de 2,3-dihydro-1-(1H-imidazol-4-yl)-1H-indène-5-ol au départ de 2 g de 4-(2,3-dihydro-5-méthoxy-1H-indén-1-yl)-1H-imidazole préparé à l'exemple 2.6.b.
P.F.: 170°C.

| Analyse pour $C_{12}H_{12}N_2O$.HCl en %: | | | | |
|---|---|---|---|---|
| calculé: | C 60,88 | H 5,53 | N 11,83 | $Cl^-$ 14,98 |
| trouvé: | 60,99 | 5,60 | 11,55 | 14,46 |

**Revendications**

**1.** 4-(1,2,3,4-tétrahydro-1-naphtalényl)-1H-imidazoles et 4-(2,3-dihydro-lH-indén-1-yl)-lH-imidazoles substitués, leurs isomères optiques et leurs mélanges racémiques, répondant à la formule générale

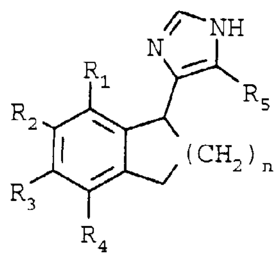

(I)

dans laquelle

n = 1 ou 2,
$R_1$, $R_2$, $R_3$ et $R_4$, qui peuvent être identiques ou différents, représentent chacun un atome d'hydrogène ou d'halogène, un groupe hydroxyle, un radical alkyle ou alkoxy, et
$R_5$ représente un atome d'hydrogène ou un radical alkyle, avec la restriction que $R_1$, $R_2$, $R_3$, $R_4$ et $R_5$ ne peuvent être simultanément de l'hydrogène lorsque n est égal à 2,

les radicaux alkyle et alkoxy ayant de 1 à 4 atomes de carbone, ainsi que leurs sels d'addition d'acides non toxiques pharmaceutiquement acceptables.

**2.** (+)-4-(1,2,3,4-tétrahydro-6-méthoxy-1-naphtalényl)-1H-imidazole et ses sels d'addition d'acides non toxiques pharmaceutiquement acceptables.

**3.** 4-(1,2,3,4-tétrahydro-5-méthyl-1-naphtalényl)-1H-imidazole et ses sels d'addition d'acides non toxiques pharmaceutiquement acceptables.

**4.** 4-(2,3-dihydro-5-méthoxy-1H-indén-1-yl)-1H-imidazole et ses sels d'addition d'acides non toxiques pharmaceutiquement acceptables.

**5.** (+)-5,6,7,8-tétrahydro-5-(1H-imidazol-4-yl)-2-naphtalénol et ses sels d'addition d'acides non toxiques pharmaceutiquement acceptables.

**6.** 4-(1,2,3,4-tétrahydro-5-méthoxy-1-naphtalényl)-1H-imidazole et ses sels d'addition d'acides non toxiques pharmaceutiquement acceptables.

**7.** 5,6,7,8-tétrahydro-5-(1H-imidazol-4-yl)-1-naphtalénol et ses sels d'addition d'acides non toxiques pharmaceutiquement acceptables.

**8.** 4-(1,2,3,4-tétrahydro-6-méthyl-1-naphtalényl)-1H-imidazole et ses sels d'addition d'acides non toxiques pharmaceutiquement acceptables.

**9.** 2,3-dihydro-1-(1H-imidazol-4-yl)-1H-indène-5-ol et ses sels d'addition d'acides non toxiques pharmaceutiquement acceptables.

**10.** 4-(1,2,3,4-tétrahydro-8-méthoxy-1-naphtalényl)-1H-imidazole et ses sels d'addition d'acides non toxiques pharmaceutiquement acceptables.

**11.** 4-(1,2,3,4-tétrahydro-6,7-diméthoxy-1-naphtalényl)-1H-imidazole et ses sels d'addition d'acides non toxiques pharmaceutiquement acceptables.

**12.** 5,6,7,8-tétrahydro-5-(1H-imidazol-4-yl)-2,3-naphtalènediol et ses sels d'addition d'acides non toxiques pharmaceutiquement acceptables.

**13.** Procédé de préparation de 4-(1,2,3,4-tétrahydro-1-naphtalényl)-1H-imidazoles, de 4-(2,3-dihydro-1H-indén-1-yl)-1H-imidazoles et de leurs sels tels que définis dans la revendication 1, caractérisé en ce que

a) pour préparer les 4-(1,2,3,4-tétrahydro-1-naphtalényl)-1H-imidazoles répondant à la formule générale I

dans laquelle n = 2 et $R_1$, $R_2$, $R_3$ et $R_4$ représentent chacun un atome d'hydrogène ou d'halogène, un radical alkyle ou alkoxy, les radicaux alkyle et alkoxy ayant de 1 à 4 atomes de carbone, on cyclise un 4-phényl-1-(1-triphénylméthyl-1H-imidazol-4-yl)-1-butanol de formule

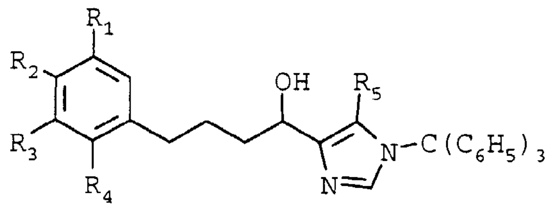

(II)

dans laquelle $R_1$, $R_2$, $R_3$ et $R_4$ ont la signification donnée ci-dessus et $R_5$ représente un atome d'hydrogène ou un radical alkyle ayant de 1 à 4 atomes de carbone, ou

b) pour préparer les 4-(2,3-dihydro-1H-indén-1-yl)-1H-imidazoles substitués répondant à la formule générale I dans laquelle n = 1 et $R_1$, $R_2$, $R_3$ et $R_4$ représentent chacun un atome d'hydrogène ou d'halogène, un radical alkyle ou alkoxy, les radicaux alkyle et alkoxy ayant de 1 à 4 atomes de carbone, on soumet un 4-(1H-indén-3-yl)-1H-imidazole de formule

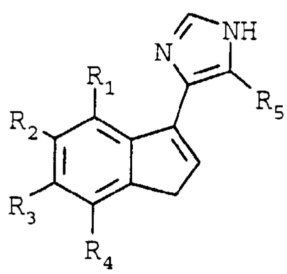

(III)

dans laquelle $R_1$, $R_2$, $R_3$ et $R_4$ ont la signification donnée ci-dessus et $R_5$ représente un atome d'hydrogène ou un radical alkyle ayant de 1 à 4 atomes de carbone, à une hydrogénation catalytique par de l'hydrogène moléculaire; ou

c) pour préparer les 1H-imidazoles substitués répondant à la formule générale I dans laquelle au moins un des symboles $R_1$, $R_2$, $R_3$ et $R_4$ représente un groupe hydroxyle, on désalkyle sélectivement le (ou les) groupe (s) alkoxy du 1H-imidazole substitué correspondant de formule

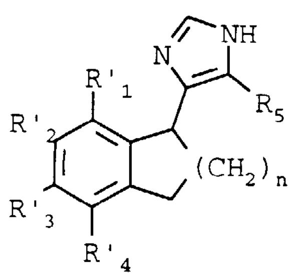

(IV)

dans laquelle n=1 ou 2 et $R_1'$, $R_2'$, $R_3'$ et $R'_4$ représentent un atome d'hydrogène ou d'halogène, un radical alkyle ou alkoxy, les radicaux alkyle et alkoxy ayant de 1 à 4 atomes de carbone, au moins un des symboles $R_1'$, $R_2'$, $R_3'$ et $R'_4$ étant un radical alkoxy et $R_5$ a la signification donnée à la revendication 1; et

d) en ce que éventuellement, on convertit les 1H-imidazoles substitués de formule I ainsi obtenus en leurs sels d'addition d'acides non toxiques pharmaceutiquement acceptables.

**14.** 1H-imidazoles substitués selon l'une quelconque des revendications 1 à 12 ou l'un de leurs sels d'addition d'acides non toxiques pharmaceutiquement acceptables pour utilisation en thérapie comme agents anti-ischémiques et anti-hypertenseurs.

**15.** Compositions thérapeutiques renfermant une quantité thérapeutiquement efficace d'un 1H-imidazole substitué

selon l'une quelconque des revendications 1 à 12 ou d'un de ses sels d'addition d'acides non toxiques pharmaceutiquement acceptables, en association avec des excipients pharmaceutiques solides ou liquides.

Office européen des brevets

# RAPPORT DE RECHERCHE EUROPEENNE

Numero de la demande
EP 95 87 0132

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | EP-A-0 269 599 (UCB, S.A.) 1 Juin 1988<br>* le document en entier *<br>--- | 1-15 | C07D233/54<br>A61K31/415 |
| A | GB-A-2 206 880 (FARMOS-YTHYMA OY) 18 Janvier 1989<br>* le document en entier *<br>--- | 1-15 | |
| A | JOURNAL OF MEDICINAL CHEMISTRY,<br>vol. 35, no. 4, 21 Février 1992 WASHINGTON US,<br>pages 750-755,<br>Y. AMEMIYA ET AL. 'Synthesis and alpha-Adrenergic Activities of 2- and 4-Substituted Imidazoline and Imidazole Analogues'<br>* le document en entier *<br>--- | 1-15 | |
| A | EP-A-0 310 745 (FARMOS-YTHYMÄ OY) 12 Avril 1989<br>* page 11, composé I; page 14, composé XVIII et page 15, lignes 1-45 *<br>----- | 1-15 | |

| DOMAINES TECHNIQUES RECHERCHES (Int.Cl.6) |
|---|
| C07D |

Le présent rapport a été établi pour toutes les revendications

| Lieu de la recherche | Date d'achèvement de la recherche | Examinateur |
|---|---|---|
| LA HAYE | 8 Mars 1996 | Fink, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)